# EUROPEAN PATENT SPECIFICATION

(11) **EP 2 922 545 B1**
(45) Date of publication and mention of the grant of the patent: **21.09.2016**
(21) Application number: 13802901.2
(22) Date of filing: 20.11.2013
(51) Int. Cl.: A61K 31/4725, A61P 37/02

(54) **SAQUINAVIR-NO FOR IMMUNOMODULATION**
SAQUINAVIR-NO ZUR IMMUNMODULATION
SAQUINAVIR-NO POUR IMMUNOMODULATION

(30) Priority: 20.11.2012 US 201261728330 P
(43) Date of publication of application: 30.09.2015
(73) Proprietor: Onconox ApS, 2200 Copenhagen (DK)
(72) Inventor: NICOLETTI, Ferdinando, I-95021 Cannizzaro (CT) (IT)
(74) Representative: Bianchetti Bracco Minoja S.r.l.
(86) International application number: PCT/EP2013/074264
(87) International publication number: WO 2014/079868

(56) References cited:
- WO-A1-99/63998
- WO-A1-03/051361
- WO-A1-2010/012466
- WO-A1-2012/088305
- HOSSEINI H ET AL: "Protection against experimental autoimmune encephalomyelitis by a proteasome modulator", JOURNAL OF NEUROIMMUNOLOGY, ELSEVIER SCIENCE PUBLISHERS BV, NL, vol. 118, no. 2, 30 August 2001 (2001-08-30), pages 233-244, XP002233724, ISSN: 0165-5728, DOI: 10.1016/S0165-5728(01)00352-6
- DELMONTE ET AL: "Immunomodulatory effects of two HIV protease inhibitors, Saquinavir and Ritonavir, on lymphocytes from healthy seronegative individuals", IMMUNOLOGY LETTERS, ELSEVIER BV, NL, vol. 111, no. 2, 10 August 2007 (2007-08-10), pages 111-115, XP022192219, ISSN: 0165-2478, DOI: 10.1016/J.IMLET.2007.06.003
- PACIFICI ET AL.: "Cytokine production in saquinavir treated mice", INT. J. IMMUNOPHARMACOL., vol. 19, 1997, pages 243-248, XP055098713, cited in the application
- FILIP PETKOVIC ET AL: "Saquinavir-NO inhibits S6 kinase activity, impairs secretion of the encephalytogenic cytokines interleukin-17 and interferon-gamma and ameliorates experimental autoimmune encephalomyelitis", JOURNAL OF NEUROIMMUNOLOGY, vol. 259, no. 1-2, 1 June 2013 (2013-06-01), pages 55-65, XP055098439, ISSN: 0165-5728, DOI: 10.1016/j.jneuroim.2013.03.010

## Description

The invention refers to the nitric ester of saquinavir for use in the treatment of diseases caused by disorders of the immune system.

### Background of the invention

Saquinavir (((2*S*)-*N*-[(2*S*,3*R*)-4-[(3*S*)-3-(*tert*-butylcarbamoyl)-decahydroisoquinolin-2-iy]-3-hydroxy-1-phenylbutan-2-il]-2-(quinolin-2-ylformamido)butanediamide), abbreviated as Saq, is a human immunodeficiency virus (HIV) protease inhibitor and it is routinely used as a constituent of Highly Active Anti-Retroviral Therapy (HAART). Besides its potent anti-retroviral effects, this drug also potently affects tumor cells (Toschi et al., 2011). Still, application of Saq is limited due to its toxicity (Flexner, 1998), and it was an idea of reducing side effects while pertaining anti-cancer properties of Saq that initiated generation of nitric oxide (NO)-modified version of the drug, Saquinavir-NO (Saq-NO). This agent is produced by covalent attachment of NO to the parental drug (Maksimovic-Ivanic et al., 2009 and WO 2010 / 012466) and thorough studies have shown that it has superior anti-cancer properties against various tumors, including melanoma, astrocytoma, prostate cancer cells and various human multidrug resistant tumor cell lines in comparison to Saq, yet exerting no toxicity in mice subjected to the treatment (Maksimovic-Ivanic et al., 2009; Mijatovic et al., 2011; Rothweiler et al., 2010; Donia et al., 2010; Mojic et al., 2012). Also, Saq-NO has retained anti-HIV potency of its parental drug (Canducci et al., 2011). These studies imply that Saq-NO has a potential as anti-tumor and anti- HIV therapeutic.

### Description of the invention

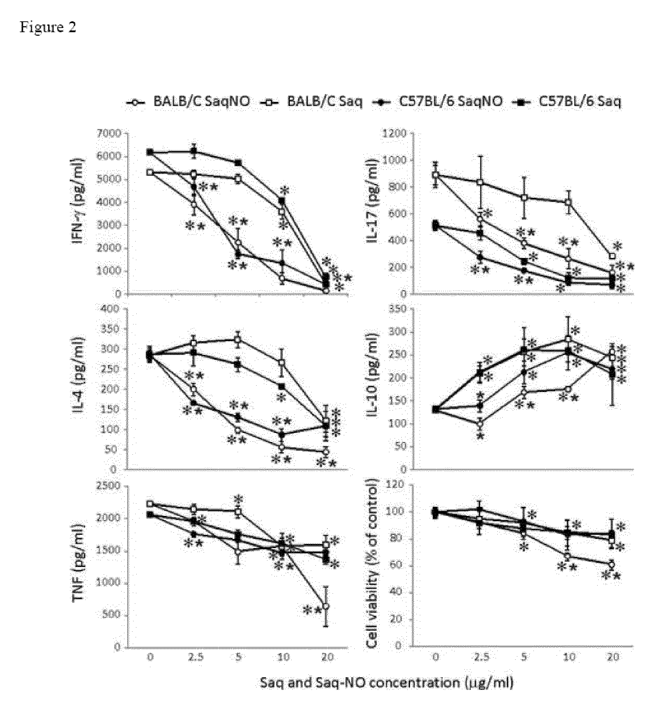

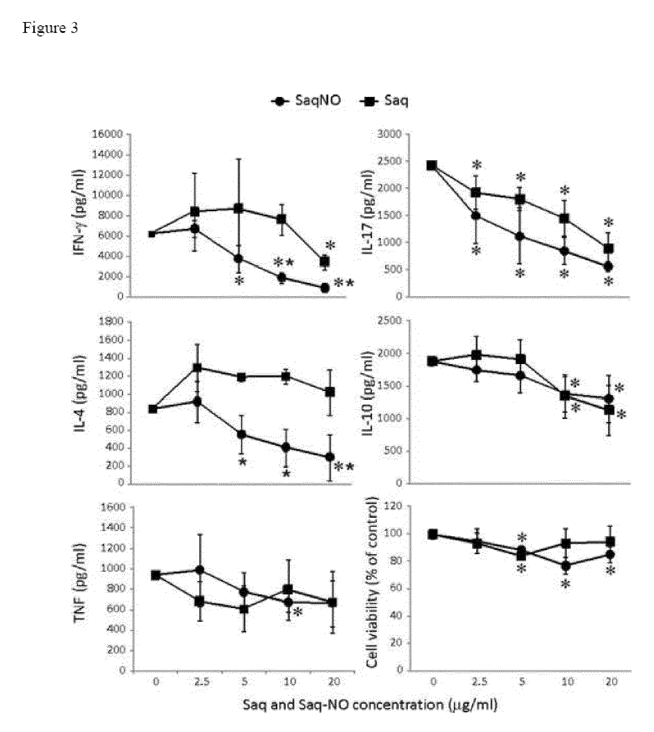

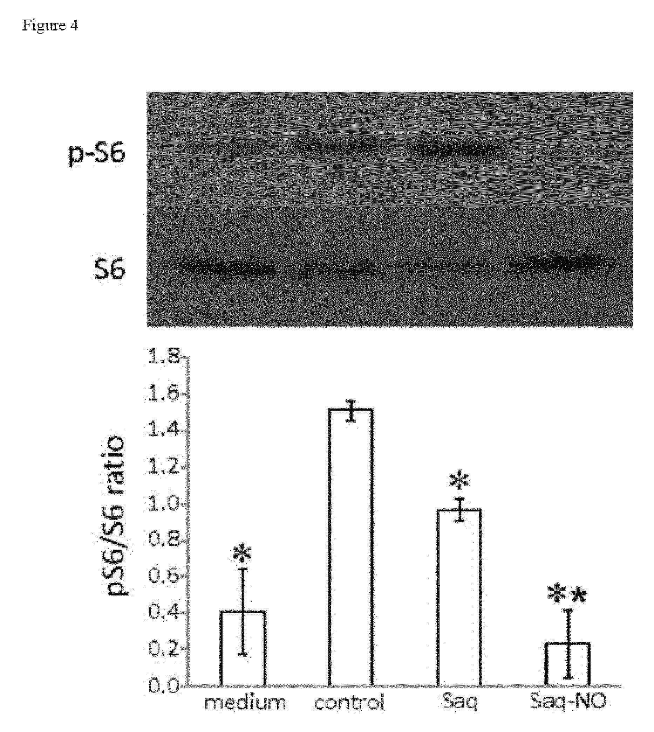

It has now been found that the nitric ester of Saquinavir having formula I, 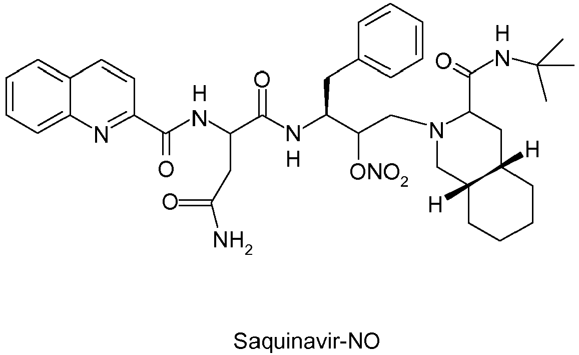 hereinafter designated as NO-saquinavir, Saq-NO or OX-1001, has interesting immunomodulatory properties, potently modulating the production of various cytokines in polyclonally-activated and antigen stimulated T cells. Saq-NO is able to modulate production of Th1, Th2, pro- and anti-inflammatory cytokines. Its effect is equally potent in polyclonally-stimulated mixed populations of immune cells (SPC and LNC) or purified T cells (CD4+ cells) and in antigen-stimulated T cells (encephalitogenic MBP-specific cells). It should be mentioned that the immunomodulatory properties of Saq have been studied upon in vitro and ex vivo conditions using peripheral blood mononuclear cells of healthy individuals (Delmonte, 2007) and spleen cells of mice previously treated with Saq (Pacifici et al.,1997) and conflicting results had been obtained. Out of the 3 cytokines studied, e.g. IL-2, IFN-gamma and TNF-alpha, in human PBMC stimulated with PMA plus ionomycin (Delmonte, 2007) Saq was found to downregulate the synthesis of IL-2, and IFN-gamma production at concentration ranging between 10 and 20 µM without influencing that of TNF-alpha. Simultaneously Saq inhibited PHA or anti-CD3-induced proliferation at concentrations ranging between 5 and 20 mM. In contrast, in the *ex vivo* study carried out in mice, spleen cells obtained from mice previously treated with Saq secreted more IFN-gamma and IL-2 than the spleens of control mice (Pacifici, 1997). Conversely IL-1 beta, IL-10 and TNF-alpha production were not modified by in vivo exposure to Saq (Pacifici, 1997). Therefore, the results of our in vitro studies indicate that NO-hybridization induce a profound immunopharmacological modification of Saq that ensues in a larger and greater degree of immunomodulation that includes in vitro inhibition of IFN-gamma, IL-17, TNF-alpha, IL-4 and IL-10 from unfractionated spleen cells and/or purified murine CD4 T cells (Figures 2 and 3). The effects of Saq-NO were stronger and of greater magnitude than those of Saq and for some cytokines already observable at the concentrations of 2.5 and 5 mM. No effects could be seen for Saq at these concentrations with the exception of an inhibitory effect on IL-17 secretion from purified CD4+ T cells (Figure 3). In addition, molecular studies revealed that Saq-NO, but not Saq, inhibited phosphorylation of S6 kinases (Fig. 4). Taken together these data demonstrate that Saq-NO is a new chemical entity endowed with different and in general stronger immunomodulatory effects than Saq.

More particularly, it has been found that Saq-NO down-regulates the production of proinflammatory cytokines involved in the pathogenesis of autoimmune disease, specifically INF-gamma, IL-17, TNF-alpha, IL-4, IL-10 and TNF generation in murine SPC, rat LNC and purified murine CD4+ cells.

Importantly, Saq-NO was able to inhibit production of IFN-g and IL-17 in MBP-specific T cells, and this finding supports the therapeutic application of Saq-NO in central nervous system (CNS) autoimmunity. IFN-g and IL-17-producing cells, Th1 and Th17 cells, respectively, are the key pathogenic populations in the pathogenesis of multiple sclerosis (MS) and its animal model experimental autoimmune encephalomyelitis (EAE) (El-behi et al., 2010; Fletcher et al., 2010; Jager et al., 2010). Importantly, Saq-NO inhibited IFN-g and IL-17 generation in ex vivo re-stimulated DLNC, but also in *in vivo* re-stimulated SCC, which implies that it is potent in restraining both maturing and mature effector Th1 and Th17 cells. Although its effect on DLNC and SCC viability was limited in comparison to its effect on IFN-g and IL-17 release, this effect also supports the therapeutic property of Saq-NO in the CNS autoimmunity, as it contributes to limitation of the CNS inflammation. Th1 and Th17 cells are also important for other organ-specific and systemic autoimmune and inflammatory disorders, including rheumatoid arthritis, respiratory inflammation, systemic lupus and allograft rejection (Afzali et al., 2007).

Saq-NO diminishes S6 phosphorylation in CD4+ T cells, while the effect of Saq is much weaker, which corresponds to their respective effects on cytokine generation in T cells. The observed reduced phosphorylation of S6 implies that Saq-NO affects S6K activation and that this effect is important for its influence on cytokine production. Indeed, S6K activity and S6 phosphorylation are relevant for cytokine generation in immune cells (Lee et al., 2010; Melino et al., 2008; Cao et al., 2008).

Saq-NO has profound immunomodulatory potency that translates in strong prophylactic and therapeutic effects in two well known models of Multiple Sclerosis, as reported in the experimental section reported below, given by way of example.

As a consequence, Saq-NO is useful for use in the treatment of organ-specific and systemic autoimmune disease wherein a dysregulation of the production of cytokines is involved.

Examples of said diseases include idiopathic Addison's disease, autoimmune hepatitis, biliary cirrhosis, primary sclerosing cholangitis, Guillain Barrè syndrome, multiple sclerosis, optical neuritis, Hashimoto's thyroiditis, psoriasis, rheumatoid arthritis, Sjogren's syndrome, systemic lupus erythematous, Type 1 diabetes mellitus and uveitis.

Saq-NO is also useful for use in the treatment of ischemia-reperfusion, graft versus host Diseases and in the prevention of graft rejection, endo and exo-toxemia and gouty arthritis.

The invention also provides pharmaceutical compositions comprising Saq-NO in admixture with suitable carrier/excipients. The compositions of the invention may be administered by any known route, particularly by the oral, parenteral, topical (including ophthalmic), transdermal, rectal route.

The dosage may vary within wide limits according to the illness to be treated, the patients weight, sex and age. It will anyhow be usually ranging from 10 to 1000 mg daily, as a single administration or divided in two or three daily administrations. Unit doses of 5-500 mg , particularly 100-250 mg, may be used.

For the treatment of uveitis, eye-drops will be a preferred administration form. The concentration of the active ingredient in the topical/ophthalmic formulations will range from 0.01 to 10%, preferably from 0.1 to 2%. Suitable excipients such as hyaluronates, tamarind seed polysaccharide and the like will be conveniently used. Alternatively, intra-vitreal administration of sterile solutions may be used. Suitable dosages will range from 0.1 to 10 mg per injection.

The dosage for other administration forms, e.g for oral or parental formulations, will be easily determined by any skilled practitioner according to the toxicological, pharmacokinetics and pharmacodynamic properties as well as according to the patients' conditions (severity of the disease and degree of advancement), weight, age and sex. The dosages will be generally similar to that already known in clinical practice for the parent compound saquinavir.

Non-toxic salts, solvates or crystalline/polymorphic forms of Saq-NO may also be used instead of Saq-NO.

### Brief Description of the Figures

Figure 1 shows the influence of Saq-NO on cytokine generation and viability of rat LNC stimulated with ConA (1 µg/ml) in the presence of various concentrations of Saq-NO.
Figure 2 shows the influence of Saq-NO and Saq on cytokine generation and viability of murine SPC.
   Isolated from BALB/c and C57BL/6 mice stimulated with ConA (2.5 µg/ml) in the presence of various concentrations of Saq or Saq-NO.
Figure 3 shows the influence of Saq-NO and Saq on cytokine generation and viability of murine CD4+ Cells purified from C57BL/6 mouse SPC stimulated with anti- CD3 and anti-CD28 (both at 1 µg/ml) in the presence of various concentrations of Saq or Saq-NO.
Figure 4 shows the influence of Saq-NO and Saq on S6 phosphorylation in murine CD4+ cells purified from C57BL/6 mouse untreated (medium) or stimulated with anti-CD3 and anti-CD28 (both at 1 µg/ml) in the absence (control) or presence of Saq or Saq-NO.
Figure 5 shows the influence of Saq-NO and Saq on IFN-g and IL-17 generation and viability of MBP specific T cells.
Figure 6 shows the effects of the late prophylactic (A) or therapeutic (B) treatment with Saq-NO on body weight increase and clinical course in MOG-induced EAE in C57B16 mice immunized with the MOG35-55 peptide in CFA and pertussis toxin, and treated under either a late prophylactic or therapeutic regime with Saq-NO 10 mg/kg, Saq or vehicle.
Figures 7 shows the effects of test compounds on the development of MOG-induced EAE in C57B16 mice.
Figure 8 shows the effects of the late prophylactic treatment with Saq-NO on body weight increase and clinical course in PLP-induced EAE in SJL mice.
Figures 9 refer to the experimental model of uveitis (see point 1.11 and 2.7):
   A/ Histological retinal sections of EAU group on day 14 after immunization, showing an architecture disruption with photoreceptor degeneration and little damage affecting all retina layers.
   B/ Histological section from a control group, showing the typical stratiform morphology and the ordered retina layers. GCL: ganglion cell layer; IPL: inner plexiform layer; OPL: outer plexiform layer; INL: inner nuclear layer; ONL: outer nuclear layer; PIS: photoreceptor inner segments; POS: photoreceptor outer segments.
   C/ Histological section from Saq-NO treated group, normal retina morphology is seen on SaqNO treated rats 14 post immunization.

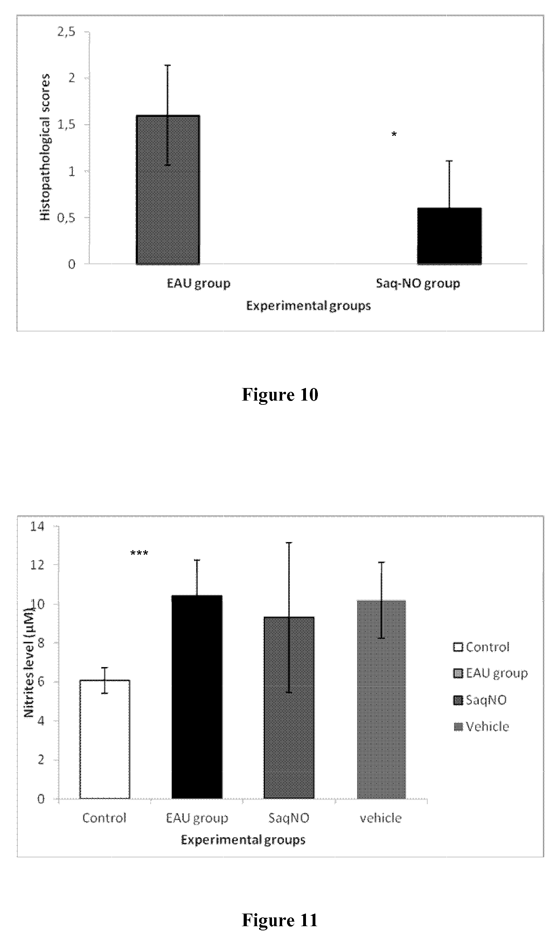

Figure 10 shows the histopathology scores in the uveitis model, treatment with 10 µg/kg of Saq-NO ameliorated the histopathological scores significantly. * P<0.05.
Figure 11 shows the Nitric oxide production *in vivo* in the uveitis model. The systemic nitrites production showed a significant elevated amounts in EAU group compared to control rats (*** p<0.001). However, treatment of rats with Saq-NO reduced systemic nitric oxide production in comparison to EAU group. P=0.51.

### Experimental section

### 1. Material and Methods

### 1.1 Reagents

RPMI-1640 medium and foetal calf serum (FCS) were from PAA Laboratories (Pasching, Austria). DMSO was from Sigma-Aldrich (St. Louis, MO). Saq was purchased from Hoffman-La Roche. Saq-NO was obtained from OncoNox (Copenhagen, Denmark) and was synthesized as described previously (Maksimovic-Ivanic et al., 2009).

### 1.2 Cells and cell cultures

Experimental animals (C57BL/6 mice, BALB/c mice and Dark Agouti rats) were obtained from the Animal House Facility of the Institute for Biological Research "Sini 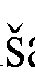 a Stamković", Belgrade, Serbia. Spleen cells (SPC) were isolated from murine spleens and lymph node cells (LNC) were obtained from rat cervical lymph nodes of untreated animals. Draining lymph node cells (DLNC) were obtained from popliteal lymph nodes 8-10 days after immunization of rats with myelin basic protein (guinea pig MBP, kind gift from professor Alexander Fluegel, University of Goettingen, Germany) and complete Freund's adjuvant (CFA, Difco, Detroit, MI). The organs were mechanically disrupted, passed through 40- µm nylon mesh filter and the resulting suspension was collected by centrifugation. Red blood cells from single cell suspensions obtained from spleens were lysed using RBC Lysis Buffer (eBioscience, San Diego, CA). CD4+ cells were purified from SPC using biotin conjugated antibody specific for CD4 (eBioscience) and IMag SAv particles plus (BD Biosciences, San Diego, CA). Obtained purity of CD4 population was more than 97% as determined by flow cytometry. SPC and LNC were seeded at 5 x 106/ml/well, DLNC at 2.5 x 106/ml/well and CD4+ cells at 1 x 106/ml/well of 24-well plates. Cells infiltrating spinal cords (SC) were obtained from SC of rats immunized with MBP+CFA at the time when rats suffered from severe clinical signs of experimental autoimmune encephalomyelitis (EAE). Rats were perfused with sterile PBS, SC were homogenized, adjusted to 30% Percoll (Sigma-Aldrich) and overlaid on 70% Percoll gradient. Following centrifugation at 870 g for 50 min the SC cells (SCC) were recovered from the 40%/70% Percoll interface and washed in RPMI-1640 medium. SCC were seeded at 0.5 x 106/200 µl/well of 96-well plates. SPC and LNC were stimulated with 2.5 µg/ml concanavalin A (ConA, Sigma-Aldrich), DLNC with MBP (10 ng/ml), CD4+ cells with anti-CD3 and anti-CD28 antibody (1 µg/ml each, both from eBioscience) and SCC were left untreated.

### 1.3 Cell viability assays

In order to assess the viability of SPC, LNC, DLNC and SCC by mitochondrial activity assay we used the mitochondrial-dependent reduction of 3-(4,5-dimethylthiazol-2-yl)-2,5-phenyltetrazolium bromide (MTT) to formazan. At the end of appropriate treatments, SPC and LNC were collected in tubes, spun down, supernatants removed and the cell pellets dissolved in 0.5 µg/ml MTT (Sigma-Aldrich) solution. Incubation with MTT lasted for 30 minutes at 37°C and cells were centrifuged once more. DMSO was added to the pellets to dissolve the formazan crystals. The absorbances in MTT assay were measured at 570540 nm, with a correction at 690 nm, using an automated microplate reader (LKB 5060-006, LKB, Vienna, Austria).

### 1.4 Detection of apoptotic cell

Detection of apoptosis among murine SPC was performed by staining of cells with Annexin V-FITC (Biotium, Hayward, CA) according to the manufacturer's suggestions. The cells positive for Annexin V-FITC were considered apoptotic. The stained cells were acquired by a FACSCalibur cytometer (BD Biosciences) and analyzed using CellQuest Software (BD Biosciences).

### 1.5 ELISA

Cytokine concentration in cell culture supernatants was determined by sandwich ELISA using MaxiSorp plates (Nunc, Rochild, Denmark) and anti-cytokine paired antibodies according to the manufacturer's instructions. Samples were analyzed in duplicate for murine/rat IL-17, murine IL-10, murine TNF, murine IL-4 (eBioscience), murine IFN-γ, rat IFN-γ, rat IL-4 (R&D, Minneapolis, MN), rat IL-10 and rat TNF (BD Biosciences). The results were calculated using standard curves made on the basis of known concentrations of the appropriate recombinant cytokines.

### 1.6 Immunoblot

Whole-cell lysates were prepared in a solution containing 62.5 mM Tris-HCl (pH 6.8, 2% w/v sodium dodecyl sulfate (SDS), 10% glycerol, 50 mM dithiothreitol (DTT), 0.01% w/v bromophenol blue, 1 mM phenylmethanesulphonyl fluoride or phenylmethylsulphonyl fluoride (PMSF), 1 µg/ml aprotinine, 2 mM EDTA and samples containing 20 µg of proteins (measured by Lowry protein assay) were electrophoresed on a 12% SDS-polyacrylamide gel. The samples were electro-transferred to polyvinylidene difluoride membranes at 5 mA/cm2, using semi-dry blotting system (Fastblot B43, Biorad, Muenchen, Germany). The blots were blocked with 5% w/v nonfat dry milk in PBS 0.1% Tween-20 and probed with specific antibodies to S6 and phosphorylated-S6 (Ser240/244) at 1:500 dilution (both from Cell Signaling Technology, Boston, MA), followed by incubation with secondary antibody at 1:10000 dilution (ECL donkey anti-rabbit horseradish peroxidase (HRP)-linked, GE Healthcare, Buckinghamshire, England, UK). Detection was performed by the chemiluminescence (ECL, GE Healthcare) and photographs were made by X-ray films (Kodak, Rochester, NY). Densitometry was performed with Scion Image Alpha 4.0.3.2 (Scion Corporation, Frederick, MD).

### 1.7 Animals

Seven to 8 weeks-old male C57B16 mice and 6 to 7 weeks-old female SJL mice (Harlan Laboratories srl, San Pietro al Natisone, Udine, Italy) were used in the experiments. They were kept under standard laboratory conditions (non specific pathogen free) with free access to food (Harlan Global Diet 2018) and water and were allowed to adapt at least one week to their environment before commencing the study.

Animals were housed within a limited access rodent facility under non specific pathogen free Conditions. Automatically controlled environmental conditions are set to maintain temperature at 20 - 24°C with a relative humidity (RH) of 30 - 70%, 10-30 air changes /hr and a natural dark:light cycle.

### 1.8 Induction of MOG-induced EAE

MOG35-55 was synthesized by Genemed synthesis (San Francisco CA). The mice were immunized with 200 µg MOG emulsified in CFA with 1 mg of *Mycobacterium tuberculosis* H37RA (Difco, Detroit, MI, USA) to make a 1:1 emulsion. Each mouse received subcutaneous injections of 200 µl emulsion divided among two sites draining into the axillary lymph nodes. Pertussis toxin (Calbiochem, Nottingham, UK) was used as a coadjuvant and was administered i.p. at the dose of 200 ng/mouse on day 0 and 2 post immunization. The mice were observed every day by measuring their body weights and clinical signs of EAE until 30 days after immunization. These clinical grading were carried out by an observer unaware of the treatment: 0 = no sign of disease; 0.5 = partial tail paralysis; 1 = tail paralysis; 1.5 = tail paralysis + partial unilateral hindlimb paralysis; 2 = tail paralysis + hindlimb weakness or partial hindlimb paralysis; 2.5 = tail paralysis + partial hindlimb paralysis (lowered pelvi); 3 = tail paralysis + complete hindlimb paralysis; 3.5 = tail paralysis + complete hindlimb paralysis + incontinence; 4 = tail paralysis + hindlimb paralysis + weakness or partial paralysis of forelimbs; 5 = moribund or dead.

The study involved 4 groups of 10 animals each. All the groups were immunized with the MOG35-55 peptide in CFA and pertuxis toxin, according to the immunization procedure described above and assigned to the following treatments:

| **Group** | **Test Substance** | **Dose** | **Administration route** | **Administration volume/rate** | **Frequency** | **Regime** |
|---|---|---|---|---|---|---|
| **1** | **Saquinavir** | 10 mg/Kg | i.p. | 10 mL/kg | Daily | Prophylactic |
| **2** | **OX1001** | 10 mg/kg | i.p. | 10 mL/kg | Daily | Prophylactic |
| **3** | **OX1001** | 10 mg/kg | i.p. | 10 mL/kg | Daily | Therapeutic |
| **4** | **Vehicle (DMSO/H2O)** | --- | i.p. | 10 mL/kg | Daily | Prophylactic |

The late prophylactic treatment started on day 7 immunization and continued until day 30; the therapeutic regime started at the onset of the disease and continued for 23 consecutive days.

### 1.9 Induction of EAE and clinical score

PLP (139-151) was synthesized by Genemed synthesis (San Francisco CA). EAE was induced as described by J. St. Louis et al. (4). The mice were immunized with 75 µg PLP emulsified in CFA with 6 mg/ml Mycobacterium tuberculosis H37RA (Difco, Detroit, MI, USA) to make a 1:1 emulsion. Each mouse received subcutaneous injections of 200 µl emulsion divided among four sites draining into the axillary and inguinal lymph nodes. Pertussis toxin (Calbiochem, Nottingham, UK) was used as a co-adjuvant and was administered i.p. at the dose of 200 g/mouse on day 0 and 2 post immunization. The mice were observed every day by measuring their body weights and clinical signs of EAE until 30 days after immunization. These clinical grading were carried out by an observer unaware of the treatment: 0 = no sign of disease; 0.5 = partial tail paralysis; 1 = tail paralysis; 1.5 = tail paralysis + partial unilateral hindlimb paralysis; 2 = tail paralysis + hindlimb weakness or partial hindlimb paralysis; 2.5 = tail paralysis + partial hindlimb paralysis (lowered pelvi); 3 = tail paralysis + complete hindlimb paralysis; 3.5 = tail paralysis + complete hindlimb paralysis + incontinence; 4 = tail paralysis + hindlimb paralysis + weakness or partial paralysis of forelimbs; 5 = moribund or dead.

Four groups of 7/8 mice were treated under a late prophylactic regime from day 7 to day 30 as follow:
**Group 1:** Saquinavir 10 mg/Kg (i.p. once daily)
**Group 2:** Saq-NO 10 mg/Kg (s.c. once daily)
**Group 3:** Saq-NO 20 mg/Kg (i.p. once daily)
**Group 4:** DMSO/H2O (vehicle) (i.p. once daily)

### 1.10 Statistical analysis

A Student's t test was performed for statistical analysis. A p value less than 0.05 was considered statistically significant.

**1.11 The effects of Saquinavir-NO (OX-1001) on the development of experimental autoimmune uveoretinitis (EAU) in the Wistar rat.**

### Material and methods:

### Animals

Female Wistar Rats n=28 (8 weeks-old) were purchased from Pasteur Institute (Algiers, Algeria). These rats were acclimated to their new environment for one week before the start of experiments and kept under normal conditions with a 12 h dark/light cycle and free access to food and water.

These animals were divided into four groups:
**Control group** (n=6) it consisted in non treated animals
**Vehicle group** (n=6) received a daily intraperitoneal injection of DMSO at 20% starting from the day of immunization.
**Retinal crude extract immunized rats** (referred to as EAU group n=8) received a single subcutaneous injection of 200 µL with Retinal Crude Extract emulsified in complete Freund's adjuvant; CFA (v/v) and were left untreated.
**SaqNO (reference) treated rats (n=8)** they received a daily intraperitoneal injection of SaqNO at 10 µg/kg starting from the day of immunization.

### Preparation of retinal crude extract:

Fresh bovine eyes (*n* =20) were used in the experiments. They were cut just in posterior to the ora serrata, the vitreous was then removed without causing retinal detachment. The retinas were removed carefully and put in PBS, pH 7.4. They were then subjected to three successive thermal shock (-20°C and + 37°) in order to release retinal antigens. The retinal crude extract was obtained after a centrifugation at 4000 rpm for 15 min and conserved at -20°C until further utilization.

### Induction of Experimental Autoimmune Uveitis (EAU):

The animals were immunized subcutaneously with 100 µl of retinal crude extract emulsified with an equal volume of complete Freund's adjuvant CFA containing 1 mg/mL *of M. tuberculosis H37Ra* without pertussis toxin, in a total volume of 200 µl. The animals were sacrificed on day 14 after immunization. In parallel, all rats from each group were also sacrificed.

### Plasma collection

Blood was collected from each experimental group by cardiac puncture in EDTA containing tubes. After centrifugation at 3000 rpm for 10 minutes, plasma was collected and stored at -45°C until nitrites determination by Griess reaction.

### Eyes collection for histological studies

For histopathological analysis, eyes were enucleated from each group and fixed in 10% phosphate-buffered formaldehyde. Tissue sections of 5 µm thickness were stained with hematoxylin and eosin (H&E). Sections were examined using a standard microscope (Zeiss) and pictures were taken using a digital camera at x40 resolution Histological criteria were based on the degree of architectural changes. The histological scores were assessed semi-quantitatively by microscopic examination as follows:
No morphological changes, 0; minimal morphological changes of the outer nuclear layer (ONL) in retina, 1; atrophy of INL, 2; disappearance of INL, 3; and damage of total retinal layers, 4.

### Nitrites level measurement:

Griess reaction was used to determine nitrites levels as indicator of NO production, in the plasma using a spectrophotometer. Briefly, 100 µl of each sample were mixed with 50 µl of Griess reagent (5% sulfanilamide, 0.5% naphthylethylenediamine dihydrochloride, and 20% HCl). These samples were incubated at room temperature for 20 min and the absorbance at 543 nm was read by spectrophotometer. The nitrites concentration was determined using a standard curve constructed with sodium nitrite (NaNO2) between (0-200 µmol/ml).

### 2 Results

### 2.1 Saq-NO inhibits generation of cytokines in rat LNC and murine SPC

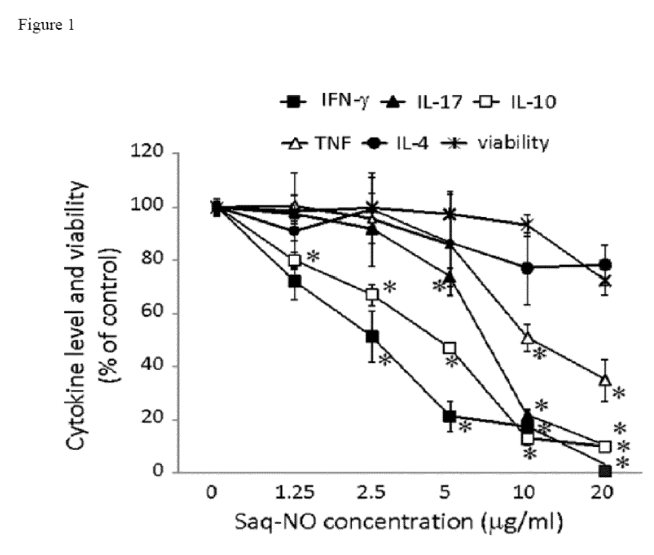

In order to investigate the potential of Saq-NO to modulate cytokine production in immune cells, rat LNC were stimulated with ConA in the presence of various concentrations of Saq-NO for 24 hours and the levels of IFN-g, IL-4, IL-17, IL-10 and TNF were determined in cell-free culture supernatants. Compared to control untreated cells, Saq-NO dose-dependently inhibited release of all of the examined cytokines, except IL-4 (Fig. 1). LNC viability was affected only with the highest dose of Saq-NO, but the difference did not reach statistically significant level in comparison to untreated control (Fig. 1). Effects of Saq-NO on the cytokine generation were examined in murine SPC, as well. Here, Saq-NO was applied in parallel with Saq and their influence on the cytokine generation and cell viability was compared. Further, two strains of mice were used, i.e. BALB/c and C57BL/6 mice which are prototypical Th2 and Th1 mice, respectively (Lohoff et al., 1998). Saq-NO had more profound effect on the release of IFN-g, IL-4, IL-17, IL-10 and TNF than Saq and the influence was undistinguishable between BALB/c and C57BL/6 mice (Fig. 2). Although Saq-NO statistically significantly inhibited SPC viability, as determined by mitochondrial activity assay, the effect was minor in comparison to the observed influence on the cytokines' generation. Moreover, the influence of Saq-NO on the cytokines was evident with concentrations of the agent that had no effect on SPC viability (Fig. 2). Proapoptotic influence of Saq-NO and Saq on SPC, measured by AnnexinV staining and cytofluorimetry, was similar and statistically insignificant even in the highest drug dose applied (Table).

**Table. Apoptosis in murine SPC treated with Saq-NO and Saq.**

| % of AnnV+ cells | | |
|---|---|---|
| C µg/ml) | Saq-NO | Saq |
| 0 | 28.9 +/- 6.9 | 28.9 +/- 6.9 |
| 5 | 30.2 +/- 7.5 | 27.5 +/- 4.8 |
| 10 | 38.4 +/- 4.8 | 31.6 +/- 6.4 |
| 20 | 44.5 +/- 11.5 | 49.8 +/- 4.8* |

Interestingly, while Saq-NO inhibited production of IFN-g, IL-4, IL-17, TNF, it stimulated production of IL-10 in murine SPC. These results imply that Saq-NO potently influences cytokine generation in immune cells. Although it predominantly has immunosuppressive effect, there are also species- and cytokine-specific effects of the agent.

### 2.2 Saq-NO inhibits production of cytokines in murine CD4+ cells

CD4+ cells were purified from C57BL/6 SPC and stimulated with anti-CD3 and anti-CD28 antibodies in the presence of various concentrations of Saq-NO for 24 hours. The influence of Saq-NO on cytokine generation was compared to its influence on CD4+ cell viability and to the influence of Saq on the same parameters. Saq-NO inhibited generation of IFN-g, IL-4 and IL-17 in CD4+ cells more effectively than Saq (Fig. 3). Influence of Saq-NO and Saq on IL-10 was similarly potent, while both agents did not modulate TNF release (Fig. 3). Saq-NO statistically significantly inhibited cell viability, yet the effect was less than its effect on cytokine generation. Thus, Saq-NO directly affects T cell cytokine production.

### 2.3 Saq-NO inhibits S6 phosphorylation

In an attempt to determine intracellular signaling that might be responsible for the observed influence of Saq-NO on the cytokines generation, level of S6 phosphorylation as the measure of S6 kinase activity was detected by immunoblot. CD4+ cells were stimulated with anti-CD3 and anti-CD28 in the presence of Saq-NO or Saq for 24 hours and cell lysates were subsequently obtained and analyzed. Saq-NO, but not Saq, diminished S6 phosphorylation in CD4+ cells (Fig. 4), thus suggesting that S6 kinase activity might be Saq-NO-specific, cytokine generation-related target in T cells.

### 2.4 Saq-NO inhibits MBP-specific production of cytokines IFN-g and IL-17

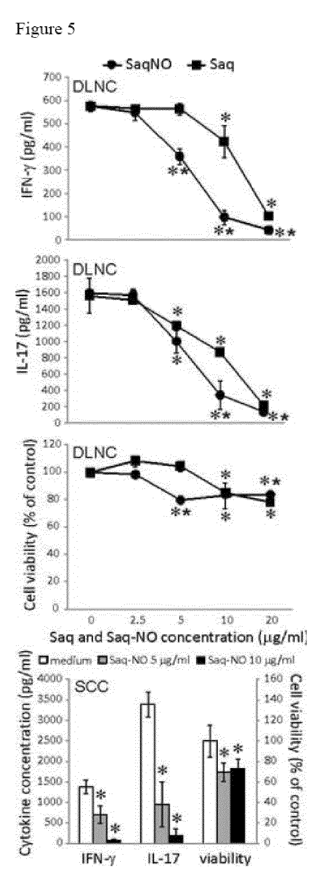

Next, we determined the influence of Saq-NO on encephalitogen-specific generation of IFN-g and IL-17. Rats were immunized with MBP+CFA and DLNC and SCC were isolated from the animals at the inductive phase and at the peak of EAE, respectively. DLNC were restimulated in vitro with MBP whereas SCC were unstimulated and both cell populations were incubated in the presence of various concentrations of Saq-NO or Saq. As the result, Saq-NO inhibited IFN-g and IL-17 release to greater extent than Saq in DLNC (Fig. 5). Saq-NO also influenced DLNC viability, but the effect was much less than its influence on IFN-g and IL-17 production (Fig. 5).

Saq-NO also potently inhibited IFN-g and IL-17 release from SCC (Fig. 5). Thus, Saq-NO efficiently modulated an antigen-specific cytokine generation of T cells.

### 2.5 Effect of test compounds on the development of MOG-induced EAE in C57B16 mice

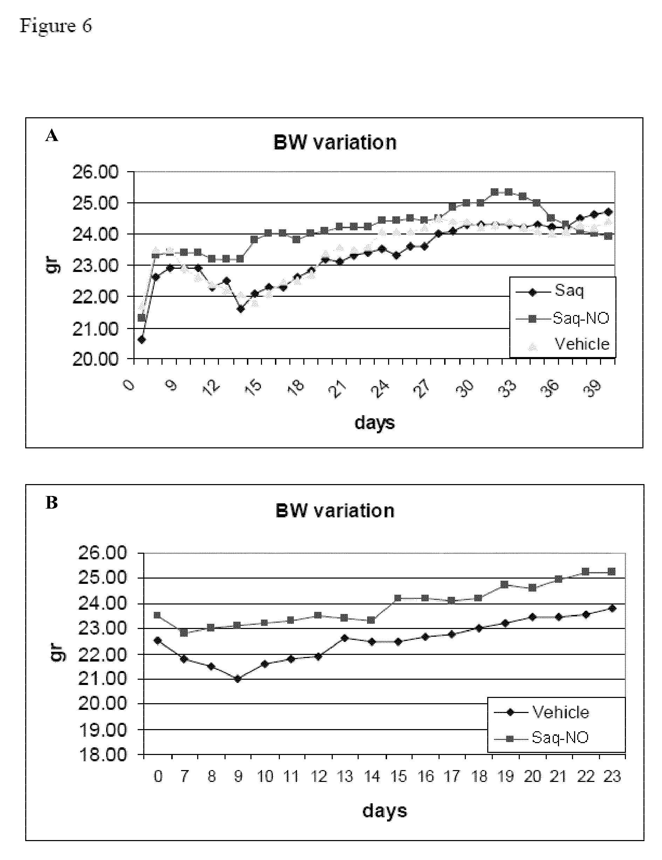

The prolonged intraperitoneal treatment with test compounds appeared to be well tolerated as judged by clinical status of the mice. The body weight variations are attributed to the clinical score of the disease (Fig. 6A-B).

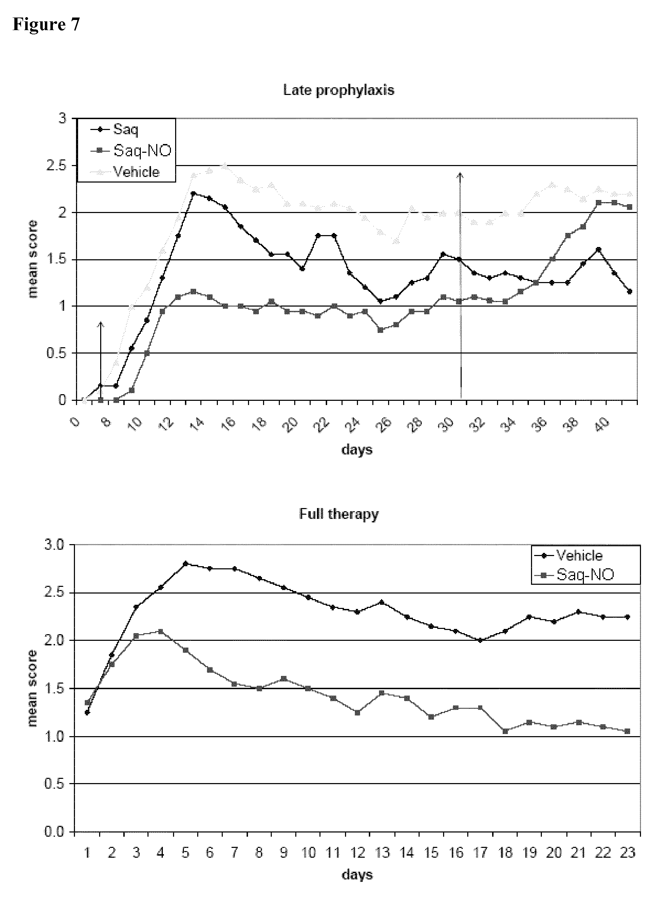

The MOG-EAE model worked well. Classical signs of EAE appeared in the vehicles-treated controls within 12 days after the immunization, with classical progressive signs of EAE (see Figs 7A-B).

The late prophylactic treatment with Saq-NO at the dose of 10 mg/Kg ameliorated the clinical course of the disease, the so treated mice exhibited a significantly lower cumulative score and duration of the disease compared to vehicle treated mice (Fig. 7A). Also the mice treated with Saq-NO at the dose of 10 mg/Kg under full therapeutic regime showed to ameliorate the clinical course of the disease exhibiting significant reduction of cumulative score and duration of the disease compared to vehicle treated mice (Fig. 7B). In contrast, the treatment with saquinavir slightly reduced the course of the disease compared to controls. At the interruption of the late prophylactic treatment, mice were observed for further 11 days, the vehicle and Saquinavir treated mice maintained the same severity of the disease, the mice treated with Saq-NO, developed a strong disease reaching, within 9 days after the treatment interruption, the clinical score of vehicle treated mice (Figs 7A-B).

### 2.6 Effect of test compounds on the development of PLP-induced EAE in SJL mice

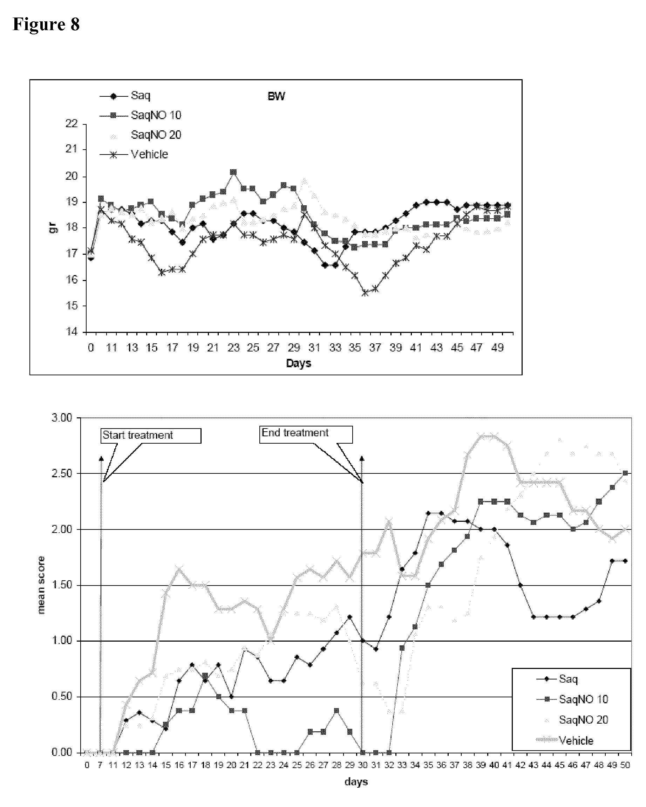

The prolonged intraperitoneal treatment with test compounds appeared to be well tolerated as judged by clinical status of the mice. The body weight variations are attributed to the clinical score of the disease (Fig. 8A).

The PLP-EAE model worked well. Classical signs of EAE appeared in the vehicle-treated controls within 15 days after the immunization, with classical progressive signs of EAE followed by relapses and remissions through the course of the study (see Fig. 8B).

The late prophylactic treatment with SaqNO at the dose of 10 mg/Kg ameliorated the clinical course of the disease, the so treated mice exhibited significantly lower cumulative score and duration of the disease compared to vehicle treated mice (Fig. 8B). The mice treated with the high dose of Saq-NO (20 mg/Kg) and with Saquinavir exhibited a milder course of the disease with a lower but not significant cum score and duration compared to vehicle-treated mice. At the interruption of the treatment, the mice were observed for further 20 days. While the vehicle treated mice continued the relapsing remitting course of the disease, the mice treated with all tested compounds, and most evidently the mice treated with Saq-NO at the dose of 10 mg/Kg, developed a strong disease reaching, already within 5 days after the treatment interruption, the clinical score of vehicle treated mice (Fig. 8B).

### 2.7 Administration of Saq-NO ameliorated the experimental autoimmune uveitis:

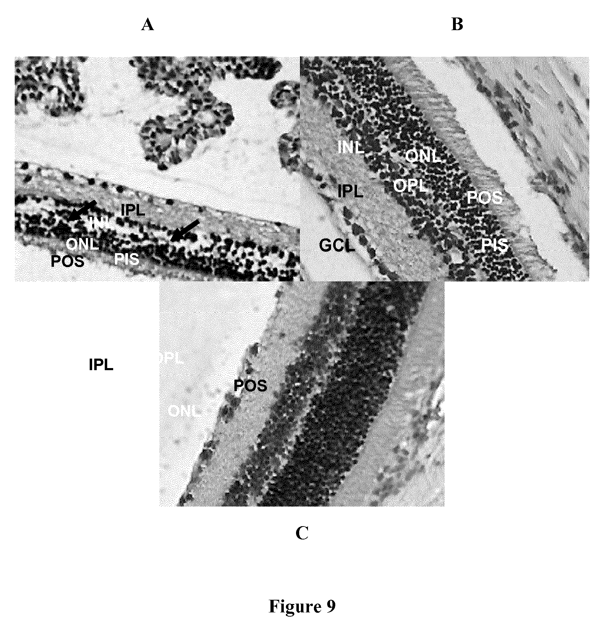

A major architecture disruption of retinal tissue characterized by photoreceptor degeneration and damage affecting all retina layers was observed in rats immunized with retinal antigen that were left untreated (Figure 9). Superimposable histological alterations were observed in the group of immunized rats that were treated with the vehicle (data not shown). In contrast, administration of Saq-NO ameliorated significantly the histological structure showing a stratified appearance of retinal tissue which is close to that observed in control rats (Figure 9).

Treatment with Saq-NO (10 µg/kg, daily), improved significantly uveitis clinical score and ameliorated the pathological manifestations of the disease. (P<0.05) (Fig. 10).

### 2.8 Administration of Saq-NO does not influence circulating nitrite levels during EAU:

Nitric oxide was assessed in vivo in all groups by measuring its end metabolite: nitrites. The results showed a significant high production of nitrites during induced EAU, in comparison to control animals (6.08±0.64 *versus* 10.42±1.84). The daily administration of 10 µg/kg of Saq-NO reduced slightly, although not significantly, reduced the circulating levels of nitrites NO as compared to EAU group (Fig. 11).

These data demonstrate that prophylactic treatment with OX-1001 (Saq-No) prevents development of EAU in Wistar rats. These data warrant the use of OX-1001 (Saq-NO) as a novel drug for the treatment of several forms immunoinflammatory uveitis in humans.

### References

Afzali, B. et al., 2007. The role of T helper 17 (Th17) and regulatory T cells (Treg) in human organ transplantation and autoimmune disease. Clin. Exp. Immunol. 148, 32-46.
Badley, A.D. et al., 1998. In vivo analysis of FAS/FasL interactions in HIV-infected patients. J. Clin. Invest. 102, 79- 87.
Canducci et al., 2011.The new and less toxic protease inhibitor saquinavir-NO maintains anti-HIV-1 properties in vitro indistinguishable from those of the parental compound saquinavir. Antiviral Res. 91, 292-295.
Cao, W. et al., 2008. Toll-like receptor-mediated induction of type I interferon in plasmacytoid dendritic cells requires the rapamycin-sensitive PI(3)K-mTOR-p70S6K pathway. Nat. Immunol. 9, 1157-1164.
Delmonte O.M.et al., Immunology Letters 2007, 111: 111-115.
Donia, M. et al., 2011.In vitro and in vivo anticancer action of Saquinavir-NO, a novel nitric oxide derivative of the protease inhibitor saquinavir, on hormone resistant prostate cancer cells. Cell Cycle 10, 492-499.
El-behi et al., 2010. Current views on the roles of Th1 and Th17 cells in experimental autoimmune encephalomyelitis. J. Neuroimmune Pharmacol. 5, 189-197.
Fletcher et al., 2010. T cells in multiple sclerosis and experimental autoimmune encephalomyelitis. Clin. Exp. Immunol. 162, 1-11.
Flexner, C., 1998. HIV-protease inhibitors. N. Engl. J. Med. 338, 1281-1292. Jäger, A., Kuchroo, V.K., 2010. Effector and regulatory T-cell subsets in autoimmunity and tissue inflammation. Scand. J. Immunol. 72, 173-184.
Kumar et al., 1999. Sustained suppression of plasma HIV RNA is associated with an increase in the production of mitogeninduced MIP-1alpha and MIP-1beta. AIDS Res. Hum. Retroviruses 15, 1073- 1077.
Lee, P.S. et al.,2010. mTORC1-S6K activation by endotoxin contributes to cytokine upregulation and early lethality in animals. PLoS One 5, e14399. Lohoff, M et al., 1998. The Th1/Th2 paradigm and experimental murine leishmaniasis. Int. Arch. Allergy Immunol. 115, 191-202.
Maksimovic-Ivanic et al., 2009.The antitumor properties of a nontoxic, nitric oxide-modified version of saquinavir are independent of Akt. Mol. Cancer Ther. 8, 1169-1178.
Melino M., et al., 2008. The effect of the JNK inhibitor, JIP peptide, on human T lymphocyte proliferation and cytokine production. J. Immunol. 181, 7300- 7306. Mijatovic et al., 2011.Cytotoxic and immune-sensitizing properties of nitric oxide-modified Saquinavir in iNOSpositive human melanoma cells. J. Cell Physiol. 226, 1803-1812.
Pacifici et al., 1997.Cytokine production in saquinavir treated mice. Int. J. Immunopharmacol. 19, 243-248.
Rothweiler F. et al., J. Jr., 2010. Anticancer effects of the nitric oxidemodified saquinavir derivative saquinavir-NO against multidrug-resistant cancer cells. Neoplasia 12, 1023-1030.
Toschi E. et al., 2011. Human immunodeficiency virus protease inhibitors reduce the growth of human tumors via a proteasome-independent block of angiogenesis and matrix metalloproteinases. Int. J. Cancer. 128, 82-93.

## Claims

1. The nitric ester of Saquinavir, of its non-toxic salts, solvates or crystalline/polymorphic forms, for use in the treatment of autoimmune diseases.

2. The nitric ester of Saquinavir for the use according to claim 1 wherein the autoimmune diseases respond to down-regulation of the production of proinflammatory cytokines.

3. The nitric ester of Saquinavir for the use according to claim 2, wherein said proinflammatory cytokines are INF-gamma, IL-17, TNF-alpha, IL-10.

4. The nitric ester of Saquinavir for the use according to claims 1-3 wherein the autoimmune diseases are selected from idiopathic Addison's disease, autoimmune hepatitis, biliary cirrhosis, primary sclerosing cholangitis, Guillain Barrè syndrome, Hashimoto's thyroiditis, psoriasis, rheumatoid arthritis, Sjogren's syndrome, systemic lupus erythematous, multiple sclerosis, optical neuritis, Type 1 diabetes mellitus and uveitis, ischemia-reperfusion, graft versus host diseases, graft rejection, endo and exo-toxemia and gouty arthritis.

5. The nitric ester of Saquinavir for the use according to claims 1-3 wherein the autoimmune disease is uveitis.

6. Topical ophthalmic formulations comprising the nitric ester of Saquinavir as an active ingredient in admixture with a suitable carrier.

## Patentansprüche

1. Salpetersäureester von Saquinavir, seinen nicht-toxischen Salzen, Solvaten oder kristallinen/polymorphen Formen zur Verwendung bei der Behandlung von Autoimmunerkrankungen.

2. Salpetersäureester von Saquinavir zur Verwendung gemäß Anspruch 1, wobei die Autoimmunerkrankungen auf eine Herabregulierung der Produktion proinflammatorischer Zytokine an-sprechen.

3. Salpetersäureester von Saquinavir zur Verwendung gemäß Anspruch 2, wobei die proinflammatorischen Zytokine INF-gamma, IL-17, TNF-alpha, IL-10 sind.

4. Salpetersäureester von Saquinavir zur Verwendung gemäß den Ansprüchen 1 bis 3, wobei die Autoimmunerkrankungen aus idiopathischer Addison-Erkrankung, autoimmuner Hepatitis, biliärer Leberzirrhose, primärer sklerosierender Cholangitis, Guillain-Barrè-Syndrom, Hashimoto-Syndrom, Psoriasis, rheumatoider Arthritis, Sjogren-Syndrom, systemischem Lupus erythematodes, multipler Sklerose, optischer Neuritis, Typ-1-Diabetes mellitus und Uveitis, Ischämie-Reperfusion, Graft-versus-Host-Erkrankungen, Transplantatabstoßung, Endo- und Exotoxemie und Gichtarthritis ausgewählt sind.

5. Salpetersäureester von Saquinavir zur Verwendung gemäß den Ansprüchen 1 bis 3, wobei die Autoimmunerkrankung Uveitis ist.

6. Topische ophthalmische Formulierungen, die den Salpetersäureester von Saquinavir als aktives Ingrediens im Gemisch mit einem geeigneten Träger umfassen.

## Revendications

1. Ester nitrique de saquinavir, de ses sels, solvates ou formes cristallines/polymorphes non toxiques, pour une utilisation dans le traitement de maladies auto-immunes.

2. Ester nitrique de saquinavir pour l'utilisation selon la revendication 1, dans laquelle les maladies auto-immunes répondent à une régulation négative de la production de cytokines pro-inflammatoires.

3. Ester nitrique de saquinavir pour l'utilisation selon la revendication 2, dans laquelle lesdites cytokines pro-inflammatoires sont l'INF-gamma, l'IL-17, le TNF-alpha, l'IL-10.

4. Ester nitrique de saquinavir pour l'utilisation selon les revendications 1 à 3, dans laquelle les maladies auto-immunes sont choisies parmi la maladie idiopathique d'Addison, l'hépatite auto-immune, la cirrhose biliaire, la cholangite sclérosante primitive, le syndrome de Guillain-Barré, la thyroïdite de Hashimoto, le psoriasis, la polyarthrite rhumatoïde, le syndrome de Sjogren, le lupus érythémateux disséminé, la sclérose en plaques, la névrite optique, le diabète sucré de type 1 et une uvéite, une ischémie-reperfusion, les maladies du greffon contre l'hôte, le rejet de greffe, une endo et exo-toxémie et l'arthrite goutteuse.

5. Ester nitrique de saquinavir pour l'utilisation selon les revendications 1 à 3, dans laquelle la maladie auto-immune est une uvéite.

6. Formulations ophtalmiques topiques comprenant l'ester nitrique de saquinavir en tant que principe actif en mélange avec un support approprié.
